# FASCICULE DE BREVET EUROPEEN

(11) **EP 3 041 487 B1**
(45) Date de publication et mention de la délivrance du brevet: **06.03.2019**
(21) Numéro de dépôt: 14739875.4
(22) Date de dépôt: 11.06.2014
(51) Int. Cl.: A61K 35/64, A23L 21/20, A61P 3/04, A61P 3/10, A61P 3/00, A61K 36/28, A61K 36/76

(54) **UTILISATION DE PROPOLIS POUR LUTTER CONTRE LES PATHOLOGIES ASSOCIEES A L'OBESITE**
VERWENDUNG VON PROPOLIS GEGEN FETTLEIBIGKEIT VERBUNDENEN PATHOLOGIEN
USE OF PROPOLIS AGAINST OBESITY-RELATED PATHOLOGIES

(30) Priorité: 11.06.2013 FR 1355357
(43) Date de publication de la demande: 13.07.2016
(73) Titulaire: Pollenergie, 47450 Saint-Hilaire-de-Lusignan (FR)
(72) Inventeur: CARDINAULT, Nicolas, F-47000 Agen (FR)
(74) Mandataire: Aquinov
(86) Numéro de dépôt international: PCT/FR2014/051408
(87) Numéro de publication internationale: WO 2014/199077

(56) Documents cités:
- CN-A- 101 828 660
- CN-A- 102 380 051
- JP-A- 2004 043 375
- JP-A- 2006 028 174
- JP-A- 2008 050 301
- KR-A- 20080 012 457
- DATABASE WPI Week 201034 Thomson Scientific, London, GB; AN 2010-F21040 XP002714272, & CN 101 700 324 A (ZHANG K) 5 mai 2010 (2010-05-05)
- IIO A ET AL: "Ethanolic extracts of Brazilian red propolis promote adipocyte differentiation through PPARgamma activation", PHYTOMEDICINE, GUSTAV FISCHER VERLAG, STUTTGART, DE, vol. 17, no. 12, 1 octobre 2010 (2010-10-01), pages 974-979, XP027286179, ISSN: 0944-7113 [extrait le 2010-04-10]
- SATOMI KOYA-MIYATA ET AL: "Propolis Prevents Diet-Induced Hyperlipidemia and Mitigates Weight Gain in Diet-Induced Obesity in Mice", BIOLOGICAL & PHARMACEUTICAL BULLETIN, vol. 32, no. 12, 1 janvier 2009 (2009-01-01), pages 2022-2028, XP055082156, ISSN: 0918-6158, DOI: 10.1248/bpb.32.2022
- SUN-SIL CHOI ET AL: "Artepillin C, as a PPAR ligand, enhances adipocyte differentiation and glucose uptake in 3T3-L1 cells", BIOCHEMICAL PHARMACOLOGY, ELSEVIER, vol. 81, no. 7, 4 janvier 2011 (2011-01-04), pages 925-933, XP028166496, ISSN: 0006-2952, DOI: 10.1016/J.BCP.2011.01.002 [extrait le 2011-01-08]
- WATARU AOI ET AL: "Improvement of insulin resistance, blood pressure and interstitial pH in early developmental stage of insulin resistance in OLETF rats by intake of propolis extracts", BIOCHEMICAL AND BIOPHYSICAL RESEARCH COMMUNICATIONS, vol. 432, no. 4, 1 mars 2013 (2013-03-01), pages 650-653, XP055082211, ISSN: 0006-291X, DOI: 10.1016/j.bbrc.2013.02.029
- YAJING LI ET AL: "Effects of Encapsulated Propolis on Blood Glycemic Control, Lipid Metabolism, and Insulin Resistance in Type 2 Diabetes Mellitus Rats", EVIDENCE-BASED COMPLEMENTARY AND ALTERNATIVE MEDICINE, vol. 4, no. 4, 1 janvier 2012 (2012-01-01) , pages 228-8, XP055082214, ISSN: 1741-427X, DOI: 10.1161/01.ATV.0000146267.71816.30
- SACHIKO JUMAN ET AL: "Caffeic Acid Phenethyl Ester Suppresses the Production of Pro-inflammatory Cytokines in Hypertrophic Adipocytes through Lipopolysaccharide-Stimulated Macrophages", BIOLOGICAL & PHARMACEUTICAL BULLETIN, vol. 35, no. 11, 1 janvier 2012 (2012-01-01), pages 1941-1946, XP055082154, ISSN: 0918-6158, DOI: 10.1248/bpb.b12-00317
- TAKEMA NAGAOKA ET AL: "Caffeic Acid Phenethyl Ester (CAPE) Analogues: Potent Nitric Oxide Inhibitors from the Netherlands Propolis", BIOLOGICAL & PHARMACEUTICAL BULLETIN, vol. 26, no. 4, 1 avril 2003 (2003-04-01), pages 487-491, XP055082145, ISSN: 0918-6158, DOI: 10.1248/bpb.26.487

## Description

L'invention est définie dans les revendications. La présente invention concerne l'utilisation d'un extrait de propolis de peuplier comprenant au moins 20% de polyphénols en poids de matière sèche comme complément alimentaire pour lutter contre les pathologies associées à l'obésité et en particulier le diabète de type II. Dans nos sociétés actuelles, la prévalence de l'obésité et du diabète de type II, est croissante et préoccupante.

Définie comme une accumulation anormale ou excessive de graisse corporelle, l'obésité peut avoir des répercussions importantes sur la santé des individus. Elle est fortement associée à l'apparition de désordres, en particulier l'insulino-résistance à l'origine du diabète de type II.

Le diabète de type II est une maladie chronique due à une sécrétion insuffisante d'insuline pour produire l'effet physiologique escompté, qui, associée à l'obésité et à la sédentarité conduit à une série de complications telles que les rétinopathies, les neuropathies, des défaillances rénales et des maladies cardiovasculaires.

L'obésité a pour cause essentielle un déséquilibre énergétique entre les calories consommées et celles dépensées. Pour les traiter, il est donc naturellement conseillé de changer de style de vie, en particulier d'adopter une alimentation moins riche en graisse et en sucres et de pratiquer une activité physique. Cette première approche est supplémentée par des traitements pharmacologiques et médicaux parfois lourds, pouvant aller jusqu'à l'intervention chirurgicale.

L'objectif de la présente invention est de proposer une approche nutritionnelle d'origine naturelle visant à limiter la survenue de pathologies associées à l'obésité induite par l'alimentation, en particulier du diabète de type II.

A cet effet, l'invention a pour objet l'utilisation d'un extrait de propolis de peuplier comprenant au moins 20% de polyphénols en poids de matière sèche pour prévenir et/ou lutter contre l'obésité et/ou le diabète de type II. En effet, de façon surprenante, l'administration de propolis permet de limiter et/ou de lutter contre la survenue des désordres associés à la prise de poids et à l'obésité.

En particulier, l'invention vise l'utilisation d'une composition comprenant au moins un extrait de propolis de peuplier comprenant au moins 20% de polyphénols en poids de matière sèche comme complément alimentaire pour prévenir et/ou limiter l'obésité et/ou le diabète de type II.

L'invention concerne également une composition particulière adaptée pour cette utilisation. La propolis est un produit fabriqué par les abeilles à partir de substances résineuses, gommeuses et balsamiques, récoltées sur les bourgeons de certains arbres et arbustes auxquelles elles y incorporent des sécrétions salivaires.

De façon surprenante, selon l'invention, la propolis présente une bonne efficacité pour prévenir et/ou lutter contre les pathologies associées à l'obésité induite par l'alimentation, en particulier le diabète de type II.

Avantageusement, l'administration à une personne obèse et/ou diabétique de type II, d'une composition comprenant au moins un extrait de propolis permet de contrôler et de limiter la survenue de désordres associés à l'obésité tels que l'inflammation à bas bruit et l'insulino-résistance.

L'invention est maintenant décrite en détail.

L'invention vise donc une composition comprenant au moins un extrait de propolis de peuplier comprenant au moins 20% de polyphénols en poids de matière sèche, pour son application comme complément alimentaire pour prévenir et/ou lutter contre les pathologies associées à l'obésité, en particulier le diabète de type II.

Par complément alimentaire au sens de l'invention, on entend une denrée alimentaire dont le but est de compléter le régime alimentaire normal et qui constitue une source concentrée de nutriments et/ou d'autres substances ayant un effet nutritionnel ou physiologique, seuls ou combinés.

Par extrait de propolis on entend toute propolis récoltée transformée par un procédé d'extraction permettant d'enlever les impuretés présentes dans l'extrait brut et/ou de concentrer la propolis en un ou plusieurs de ses constituants.

L'extrait de propolis peut se présenter sous toute forme. Préférentiellement, il se présente sous forme de poudre.

L'extrait de propolis utile selon l'invention contient des polyphénols, en particulier il comprend au moins 20% de polyphénols en poids de matière sèche de l'extrait.

La propolis utilisée peut provenir de toute origine botanique identifiée. Il peut provenir de plusieurs origines botaniques si elles sont bien identifiées et caractérisées.

Très préférentiellement il s'agit de propolis de peuplier et/ou de propolis de *Baccharis* (propolis verte du Brésil), et/ou de propolis de *Dalbergia ecastophyllum* (rouge). La propolis de *Baccharis* peut être une propolis de *Baccharis Dracunculifolia.*

Lorsque l'extrait de propolis est un extrait de propolis de peuplier, il comprend préférentiellement au moins 30% de polyphénols en poids de matière sèche de l'extrait. L'extrait présent dans la composition peut être obtenu par un procédé comprenant les étapes suivantes :
- extraction de propolis,
- concentration.

L'extrait peut ensuite être reconcentré et enfin transformé.

Les différentes étapes du procédé doivent être réalisées, sans détruire les principes actifs et sans utiliser de solvants.

Selon un mode de réalisation particulièrement adapté, l'extrait utilisé selon l'invention peut être obtenu par un procédé comprenant les étapes suivantes :
- macération de propolis brute dans une solution alcoolique, et
- concentration par évaporation.

L'extrait peut ensuite être transformé en poudre.

L'extrait de propolis obtenu est incorporé dans une composition.

La composition comprenant l'extrait de propolis est utilisée comme complément alimentaire.

La composition comprend au moins un extrait de propolis, mais peut en contenir plusieurs, en particulier deux ou trois extraits d'origine botanique différente.

Préférentiellement, la composition comprend au moins un extrait choisi parmi :
- un extrait de propolis de peuplier,
- un extrait de propolis de *Baccharis* (propolis verte du Brésil),
- un extrait de propolis de *Dalbergia ecastophyllum* (rouge).

Selon un mode de réalisation adapté, la composition contient un mélange de deux de ces extraits de propolis, ou un mélange des trois extraits de propolis.

La composition selon la présente description, en plus du ou des extrait(s) de propolis, peut contenir au moins un extrait de plante. Préférentiellement il comprend un extrait de *Trigonella foenum-graecum* et/ou un extrait de *Moringa oleifera.*

La composition peut aussi contenir du resvératrol, à partir d'une forme synthétique ou d'un extrait de plante.

La composition peut également contenir des excipients connus de l'homme de l'art, tels que du kaolin ou du fibregum® ou d'autres agents texturants, d'enrobage et/ou de gastro-résistance.

Selon un mode de réalisation adapté, la composition se présente sous forme d'une poudre. La composition selon l'invention, s'agissant d'un complément alimentaire, est administrée en plus des repas. Elle peut être administrée sous différentes formes galéniques, en particulier sous forme de gélule ou de comprimé.

De façon préférée, la dose journalière de composition comprend entre 400 et 1000 mg d'extrait de propolis en poids de matière sèche. Préférentiellement, la dose journalière est répartie en 2 ou 3 prises.

La composition selon l'invention comprenant au moins un extrait de propolis est utilisée pour son application comme complément alimentaire pour prévenir et/ou lutter contre les pathologies associées à l'obésité, en particulier le diabète de type II.

Outre l'insulino-résistance ou le diabète de type II, parmi les autres pathologies associées au diabète pour lesquelles le complément alimentaire selon l'invention peut être utilisé, on peut citer notamment l'inflammation métabolique ou inflammation à bas bruit et l'athérosclérose.

L'utilisation selon l'invention permet d'agir notamment sur les principales molécules sécrétées par le tissu adipeux pendant les périodes interprandiales, en particulier sur :
- les acides gras libres,
- l'adiponectine,
- la leptine,
- les cytokines inflammatoires, et/ou
- les chimiokines.

Les acides gras libres sont normalement sécrétés par le tissu adipeux pendant les périodes interprandiales. Au cours de l'obésité, lorsque le tissu adipeux commence à devenir insulino-résistant, en partie du fait de l'hypertrophie et de l'hyperplasie du tissu adipeux qui devient hypotoxique, la lipolyse est accélérée, conduisant à la libération massive d'acides gras libres dans la circulation. Ceux-ci vont, au niveau du foie et du muscle, perturber l'action de l'insuline.

Selon l'invention, la propolis peut être utilisée pour limiter la libération d'acides gras libres dans la circulation.

L'adiponectine, synthétisée principalement par les adipocytes, circule à des concentrations élevées chez les individus minces (5 à 30mg/l de plasma). L'adiponectine augmente la sensibilité à l'insuline, affecte la production de glucose au niveau hépatique en inhibant l'expression de deux enzymes essentielles à la néoglucogenèse, et présente également des propriétés anti-inflammatoires en modulant l'expression des cytokines pro/anti-inflammatoires, en particulier celle du TNF-α. Contrairement aux autres adipokines, sa production et sa sécrétion sont diminuées chez les individus obèses présentant une insulino-résistance ou un diabète de type II.

Selon l'invention, la propolis peut être utilisée pour augmenter le taux d'adiponectine chez les individus obèses présentant une insulino-résistance ou un diabète de type II.

Comme l'adiponectine, la leptine est produite principalement par les adipocytes. Sa concentration plasmatique ainsi que son expression dans le tissu adipeux sont positivement corrélés à la sévérité de l'obésité.

Selon l'invention, la propolis peut être utilisée pour réguler le taux de leptine chez les individus obèses.

Les cytokines inflammatoires, comme le TNF-α, sont des cytokines synthétisées par de nombreux tissus dont le tissu adipeux obèse, et qui, en plus de leurs activités pro-inflammatoires bien connues, sont impliquées dans la pathogenèse de l'insulino-résistance. Avantageusement, il est connu que la propolis est capable d'inhiber les facteurs pro-inflammatoires comme le TNF-α.

Enfin, les chimiokines sont des molécules chimioattractantes notamment pour les macrophages, sécrétées par les adipocytes.

L'utilisation selon l'invention, permet de limiter le taux de chimiokine en particulier de la MCP-1 (« monocyte chemotactic protein-1), facteur clé du recrutement des macrophages dans le tissu adipocytaire, et de la MIP-1α (macrophage inflammatory protein-1α).

Avantageusement, notamment grâce à ses différentes actions, la composition selon l'invention permet de prévenir et/ou lutter l'obésité et/ou les pathologies associées, en particulier le diabète de type II.

L'invention est à présent illustrée par des exemples d'extraits et de compositions.

### Exemples d'extraits de propolis

La propolis utilisée, en particulier la propolis de peuplier, peut être obtenue par la mise en oeuvre de la technique des grilles qui permet d'obtenir une propolis avec des caractéristiques particulières adaptées à une utilisation médicale en comparaison d'une propolis obtenue par la technique de grattage. La méthode des grilles permet d'obtenir une propolis avec un taux de polyphénols plus important et un pourcentage de cire diminué.

Une fois la propolis récoltée, elle est traitée par la mise en oeuvre d'un procédé d'extraction comprenant les étapes suivantes :
- la propolis est mélangée dans un extracteur avec de l'alcool suivant un ratio de 1/2,5 à 1/5 (w/v) pendant un laps de temps déterminé,
- le mélange subit ensuite une filtration afin de ne garder en solution liquide que les principes actifs de la propolis : les polyphénols,
- une dernière clarification par décantation gravitaire peut éventuellement être réalisée si nécessaire.

Ce procédé permet l'élaboration d'un extrait liquide concentré en principes actifs.

Cet extrait liquide peut ensuite éventuellement être encore concentré en principes actifs par alcoolisation. On obtient alors un extrait mou très concentré en principes actifs de propolis. Cet extrait mou peut être transformé en poudre.

### Exemple d'extrait 1

Un exemple d'extrait de propolis obtenu par la mise en oeuvre de ce procédé est un extrait de propolis de peuplier sous forme de poudre, présentant au moins 30% de polyphénols totaux en poids de matière sèches.

Parmi les polyphénols, l'extrait comprend notamment :
- au moins 8% (±0,8%) de pinocembrine
- au moins 5% (±0,5%) de chrysine
- au moins 4% (±0,4%) de galangine, et
- au moins 1,8% (±0,18%) de CAPE,
les pourcentages étant donnés en poids de matière sèche par rapport aux polyphénols totaux présents dans l'extrait.

### Exemple d'extrait 2

Un exemple d'extrait de propolis obtenu par la mise en oeuvre de ce procédé est un extrait de propolis de *Baccharis* sous forme de poudre, présentant au moins 20% de polyphénols totaux en poids de matière sèches.

L'extrait comprend notamment au moins 10% (±1%) d'artepelline C en poids de matière sèche par rapport aux polyphénols totaux présents dans l'extrait.

### Exemple d'extrait 3

Un exemple d'extrait de propolis obtenu par la mise en oeuvre de ce procédé est un extrait de propolis rouge sous forme de poudre. L'extrait comprend notamment des isoflavonoïdes.

### Exemples de compositions

### Exemple de composition 1

Un exemple de composition utile selon l'invention comprend :
- 50 à 75% d'un extrait de propolis selon l'exemple 1, 2 ou 3
- 5 à 15% de fibregum
- 10 à 15% de silice
- 10 à 15% de kaolin

### Exemple de composition 2

Un exemple de composition utile selon l'invention comprend :
- 15 à 50% d'extrait de propolis verte du Brésil (*exemple 2*)
- 15 à 50% d'extrait de propolis de peuplier (*exemple 1*)
- 10 à 30% de fibregum
- 10 à 30% de silice
- 10 à 30% de kaolin

### Exemple de composition 3

Un exemple de composition utile selon l'invention comprend :
- 10 à 50 % d'extrait de propolis rouge (*exemple 3*),
- 10 à 50% d'extrait de propolis verte du Brésil (*exemple 2*),
- 10 à 50% d'extrait de propolis de peuplier (*exemple* 1*)* comprenant au moins 30% de polyphénols,
- 10 à 30% de fibregum,
- 10 à 30% de silice,
- 10 à 30% de kaolin.

## Revendications

1. Composition comprenant au moins un extrait de propolis de peuplier comprenant au moins 20% de polyphénols en poids de matière sèche, pour son utilisation comme complément alimentaire pour prévenir et/ou lutter contre les pathologies associées à l'obésité induite par l'alimentation.

2. Composition pour une utilisation selon la revendication 1, pour prévenir et/ou lutter contre le diabète de type II.

3. Composition pour une utilisation selon l'une des revendications précédentes, **caractérisée en ce qu'**elle comprend également au moins un extrait de plante.

4. Composition pour une utilisation selon l'une des revendications précédentes, **caractérisée en ce qu'**elle comprend également du resvératrol.

5. Composition pour une utilisation selon l'une des revendications précédentes, **caractérisée en ce que** la composition se présente sous forme de poudre.

6. Composition pour une utilisation selon l'une des revendications précédentes, pour son utilisation comme complément alimentaire administré en plus des repas.

7. Composition pour une utilisation selon l'une des revendications précédentes, **caractérisée en ce qu'**elle comprend également au moins un extrait de propolis choisis parmi :
- un extrait de propolis de *Baccharis, et*
- un extrait de propolis de *Dalbergia ecastophyllum.*

8. Composition pour une utilisation selon l'une des revendications précédentes, **caractérisée en ce qu'**elle comprend également :
- un extrait de propolis de *Baccharis, et*
- un extrait de propolis de *Dalbergia ecastophyllum.*

## Patentansprüche

1. Zusammensetzung mit wenigstens einem Extrakt aus Bienenharz von Pappeln mit wenigstens 20% an Polyphenol im Trockengewicht zu dessen Verwendung als Nahrungsergänzungsmittel zur Prävention und/oder Bekämpfung von Pathologien, die mit der durch Ernährung hervorgerufenen Fettleibigkeit zusammenhängen.

2. Zusammensetzung zu deren Verwendung nach Anspruch 1, zur Prävention und/oder Bekämpfung von Diabetes vom Typ II.

3. Zusammensetzung zu deren Verwendung nach einem der vorhergehenden Ansprüche, **dadurch gekennzeichnet, dass** diese auch wenigstens einen Pflanzenextrakt umfasst.

4. Zusammensetzung zu deren Verwendung nach einem der vorhergehenden Ansprüche, **dadurch gekennzeichnet, dass** diese auch Resveratrol umfasst.

5. Zusammensetzung zu deren Verwendung nach einem der vorhergehenden Ansprüche, **dadurch gekennzeichnet, dass** die Zusammensetzung als Pulver vorliegt.

6. Zusammensetzung zu deren Verwendung nach einem der vorhergehenden Ansprüche, zu deren Verwendung als Nahrungsergänzungsmittel, das zusätzlich zu Mahlzeiten verabreicht wird.

7. Zusammensetzung zu deren Verwendung nach einem der vorhergehenden Ansprüche, **dadurch gekennzeichnet, dass** diese auch wenigstens einen Extrakt aus Bienenharz umfasst, das ausgewählt ist aus:
- einem Extrakt aus Bienenharz von *Baccharis,* und
- einem Extrakt aus Bienenharz von *Dalbergia ecastophyllum.*

8. Zusammensetzung zu deren Verwendung nach einem der vorhergehenden Ansprüche, **dadurch gekennzeichnet, dass** diese auch aufweist:
- einen Extrakt aus Bienenharz von *Baccharis,* und
- einen Extrakt aus Bienenharz von *Dalbergia ecastophyllum.*

## Claims

1. A composition comprising at least one extract of poplar-type propolis comprising at least 20% polyphenols by weight of dry matter, for use as a food supplement for preventing and/or combatting pathologies associated with obesity caused by diet.

2. The composition for use according to claim 1, for preventing and/or combatting type 2 diabetes.

3. The composition for use according to either of the preceding claims, **characterised in that** it also comprises at least one plant extract.

4. The composition for use according to any of the preceding claims, **characterised in that** it also comprises resveratrol.

5. The composition for use according to any of the preceding claims, **characterised in that** the composition is in powder form.

6. The composition for use according to any of the preceding claims, for use as a food supplement administered in addition to meals.

7. The composition for use according to any of the preceding claims, **characterised in that** it also comprises at least one extract of propolis chosen from:
- an extract of *Baccharis* propolis, and
- an extract of *Dalbergia ecastaphyllum* propolis.

8. The composition for use according to any of the preceding claims, **characterised in that** it also comprises:
- an extract of *Baccharis* propolis, and
- an extract of *Dalbergia ecastaphyllum* propolis.
